# EUROPÄISCHE PATENTSCHRIFT

(11) **EP 1 523 354 B1**
(45) Veröffentlichungstag und Bekanntmachung des Hinweises auf die Patenterteilung: **03.09.2008**
(21) Anmeldenummer: 03771096.9
(22) Anmeldetag: 24.07.2003
(51) Int. Cl.: A61M 5/145

(54) **VERABREICHUNGSVORRICHTUNG MIT OSMOTISCHEM ANTRIEB**
ADMINISTRATION DEVICE COMPRISING AN OSMOTIC DRIVE
DISPOSITIF D'ADMINISTRATION A ENTRAINEMENT OSMOTIQUE

(30) Priorität: 24.07.2002 DE 10233622
(43) Veröffentlichungstag der Anmeldung: 20.04.2005
(73) Patentinhaber: Disetronic Licensing AG, 3401 Burgdorf (CH)
(72) Erfinder: SCHILTGES, Gilbert, CH-3422 Kirchberg (CH); REMDE, Axel, CH-3432 Luetzelflueh (CH); GAFNER-GEISER, Simone, CH-4900 Langenthal (CH); CRIS, Catalin, CH-3063 Ittigen (CH)
(74) Vertreter: Wess, Wolfgang
(86) Internationale Anmeldenummer: PCT/EP2003/008174
(87) Internationale Veröffentlichungsnummer: WO 2004/011062

(56) Entgegenhaltungen:
- WO-A-94/05354
- DE-A- 19 939 023
- US-A- 3 604 417
- US-A- 4 773 900
- US-A- 5 279 608
- US-A- 5 769 824
- US-A- 5 869 078
- US-B1- 6 287 295

## Beschreibung

Die vorliegende Erfindung betrifft eine Vorrichtung zur Verabreichung eines injizierbaren Produkts mit einem osmotischen Antrieb, insbesondere eine Infusionspumpe zur Langzeitverabreichung von Insulin oder dergleichen.

Bei vielen medizinischen oder therapeutischen Anwendungen ist ein konstanter oder variabel einstellbarer kontinuierlicher Volumenstrom eines medizinischen oder therapeutischen Wirkstoffs notwendig oder wenigstens von Vorteil gegenüber einer einmaligen Verabreichung einer großen Wirkstoffdosis. Bei einer stationären Behandlung ist es hierfür üblich, Infusionsflaschen mit einer Wirkstoffflüssigkeit oberhalb einer Injektionsstelle anzubringen, so dass die Wirkstoffflüssigkeit aufgrund der Schwerkraft über eine Schlauchverbindung zur Injektionsstelle gebracht wird. Solche Infusionsflaschen eignen sich jedoch nicht als mobile Verabreichungsvorrichtung, insbesondere nicht bei einer Langzeitverabreichung von vergleichsweise geringe Dosen.

Es wurden daher Infusionspumpen mit einem eigenen Antrieb entwickelt, durch den ein Produkt in einem Produktbehälter kontinuierlich mit Druck beaufschlagt werden kann, so dass eine kontinuierliche Abgabe des Produkts aus dem Behälter möglich ist. Aus der US 4,838,862 ist z. B. ein tragbares Infusionsgerät mit einem osmotischen Antrieb bekannt, das ein flaches Gehäuse mit mehreren übereinanderliegenden Kammern und einen seitlich angeordneten Auslass für das Produkt aufweist. Die oberste Kammer bildet eine Lösungsmittelkammer, die mit Wasser gefüllt ist und von einer mittleren Lösungskammer, die mit einer Salzlösung gefüllt ist, durch eine feste Trägerplatte, eine darunter angeordnete poröse Lage und eine unter dieser angeordneten semipermeablen Membran getrennt ist. In der festen Trägerplatte ist eine Öffnung vorgesehen, die durch eine Abdeckfolie verschlossen ist. Eine untere Produktkammer ist von der mittleren Lösungskammer durch ein elastisches Diafragma getrennt. An der Produktkammer ist der Auslass für das Produkt durch eine seitliche Öffnung in dem Gehäuse vorgesehen. Das Infusionsgerät kann z. B. mit der Bodenfläche des Gehäuses an einer Stelle am Körper eines Anwenders aufgesetzt und dadurch mitgeführt werden. Aus dem Gehäuse ragt ein Druckknopf nach oben heraus, der über der Abdeckfolie angeordnet ist und eine in Richtung der Abdeckfolie nach innen in die Wasserkammer weisende Nadel besitzt Zur Aktivierung des Infusionsgeräts wird der Druckknopf in das Gehäuse gedrückt, so dass die Nadel die Abdichtfolie durchsticht und Wasser aus der oberen Wasserkammer durch die Öffnung in der festen Trägerplatte in die poröse Lage über der semipermeablen Membran eindringt. Durch das Wasser über der semipermeablen Membran wird in der mittleren Lösungskammer ein osmotisch bedingter Druck generiert, der auf das elastische Diafragma und damit auf die Produktkammer wirkt. Durch den Druck auf die Produktkammer wird das Produkt mit einem kontinuierlichen Volumenstrom durch den Auslass verabreicht.

Bei dem bekannten Infusionsgerät wird das elastische Diafragma großflächig über der Produktkammer angeordnet, um ausreichend Druck in der Kammer zu erzeugen. Das elastische Diafragma kann sich zwar nach innen in die Produktkammer wölben, es ist jedoch damit nicht möglich, das Produkt vollständig aus der Produktkammer auszuschütten. Der Druckknopf überdeckt im Vergleich zur Gesamtfläche der Lösungsmittelkammer eine kleine Fläche. Beim Eindrücken des Knopfes wird in der Lösungsmittelkammer wenig Druck erzeugt, so dass das Lösungsmittel allein durch die Kapillarwirkung der porösen Lage über die Gesamtfläche der semipermeablen Membran verteilt wird.

Aus der US 5,279,608 ist eine osmotische Pumpe bekannt, bei der axial hintereinander eine Kammer 12 mit Lösungsmittel, eine Kammer 7 mit einer osmotischen Salzlösung und eine Produktkammer 5 mit dem zu verabreichenden Produkt angeordnet sind. Dabei sind die Kammer 12 und die Kammer 7 durch eine semipermeable Membran getrennt, die Kammer 7 von der Produktkammer durch einen axial verschiebbaren Stopfen 6. Die Kammer 12 weist nach außen hin einen weiteren axial verschiebbaren Stopfen 14 auf, mit dem sie auf der der permeablen Membran gegenüber liegenden Seite, gegenüber der Umgebung abgeschlossen ist. Der Stopfen 14 wird durch den Unterdruck, der beim Strömen des Lösungsmittels aus der Kammer 12 entsteht, in diese hineingezogen.

Aus der WO-A-94-5354 Figur 2 ist eine osmotische Pumpe bekannt, bei der eine Kammer 54 erst bei der Benutzung mit Lösungsmittel gefüllt wird. Gleichzeitig quillt ein schwammartiges Material 60 auf und füllt die ganze Kammer 54.
Ein Trennmittel und eine Auslösevorrichtung dafür sind überflüssig weil vor die Benutzung kein Lösungsmittel im Gerät ist.

Es ist eine Aufgabe der vorliegenden Erfindung, eine Vorrichtung zur Verabreichung eines injizierbaren Produkts mit einem osmotischen Antrieb zu schaffen, die als fertig einsetzbare Vorrichtung über längere Zeit gelagert werden kann, ein schnelles Auslösen der Verabreichung ermöglicht, über eine längere Zeit kontinuierlich einen konstanten oder variablen Volumenstrom von auch nur geringem Umfang sicherstellt und eine einfache und zuverlässige Handhabung gewährleistet.

Diese Aufgabe wird durch eine Vorrichtung zur Verabreichung eines injizierbaren Produkts mit den Merkmalen nach Anspruch 1 gelöst. Vorteilhafte Weiterbildungen sind Gegenstand der rückbezogenen Unteransprüche.

Demnach umfasst die Vorrichtung zur Verabreichung eines injizierbaren Produkts ein Gehäuse, in dem ein osmotischer Antrieb untergebracht ist. Vorzugsweise weist das Gehäuse eine zylinderartige längliche Form auf, so dass sich vorteilhafterweise eine Art Längsachse definieren lässt, bezüglich derer sich die einzelnen Komponenten der Verabreichungsvorrichtung anordnen lassen. Es ist auch möglich, ein Gehäuse mit einer beliebigen anderen Form auszuwählen, auch solche bei welchen die Komponenten nicht entlang einer Achse angeordnet werden. Zum Beispiel ist es möglich, ein flächiges Gehäuse zu verwenden, bei dem entlang einer Längsseite eine erfindungsgemäße Verabreichungsvorrichtung angeordnet ist. In dem übrigen nicht von der Verabreichungsvorrichtung eingenommenen Innenraum des Gehäuses können weitere Vorrichtungen untergebracht sein, z. B. eine Anzeige- oder Kontrollvorrichtung für die Verabreichung des injizierbaren Produkts.

Für den osmotischen Antrieb sind in dem Gehäuse eine erste mit porösem Material gefüllte Kammer mit einem Lösungsmittel, eine zweite Kammer mit einer Lösung und eine Produktkammer zur Aufnahme des Produkts aufgenommen. Als Lösungsmittel kann z. B. Wasser und als Lösung eine übersättigte Salzlösung verwendet werden. Es ist vorteilhaft, die Produktkammer derart auszubilden, dass sie zur Aufnahme eines Produktbehälters, wie etwa einer herkömmlichen Ampulle geeignet ist. Dadurch kann die Verabreichungsvorrichtung in einfacher Weise mit dem Produkt gefüllt werden. Zwischen der ersten und der zweiten Kammer ist eine semipermeable Membran angeordnet, die für die Moleküle der Lösung undurchlässig ist und osmotisch aktiv ist, sobald an einer Seite das Lösungsmittel und an der anderen Seite die Lösung angrenzt. Dabei wird der zum Antrieb der Verabreichungsvorrichtung notwendige osmotische Druck erzeugt.

Weiter umfasst die erfindungsgemäße Verabreichungsvorrichtung eine Auslösevorrichtung zum Auslösen des osmotischen Antriebs. Hierfür ist zwischen der ersten und der zweiten Kammer ein unpermeables Trennmittel vorgesehen, das eine der Flüssigkeiten, das Lösungsmittel oder die Lösung, von einem Kontakt mit der semipermeablen Membran trennt. Die jeweils andere Flüssigkeit kann bereits mit der semipermeablen Membran in Kontakt sein. Zum Auslösen des osmotischen Antriebs wird das unpermeable Trennmittel durchbrochen, so dass die abgetrennte Flüssigkeit mit der semipermeablen Membran in Kontakt kommen kann. Eine andere Möglichkeit, den osmotischen Antrieb auszulösen, besteht darin, das unpermeable Trennmittel herauszunehmen oder herauszuziehen. Dies kann z. B. über einen Schlitz erfolgen, welcher mit Dichtelementen versehen ist, die die Dichtigkeit sowohl bei vorhandenem als auch bei entferntem unpermeablen Trennmittel garantieren. Es ist vorteilhaft, das unpermeable Trennmittel an einer Stelle in der ersten oder der zweiten Kammer vorzusehen, an der es leicht durch die Auslösevorrichtung durchbrochen oder herausgenommen werden kann. Als unpermeables Trennmittel eignet sich z. B. eine dünne Metallfolie, z. B. aus Aluminium oder dergleichen. Durch die Auslösevorrichtung mit dem unpermeablen Trennmittel wird verhindert, dass sich der Antrieb während einer Lagerung der Verabreichungsvorrichtung in Betrieb setzt. Die Vorrichtung kann daher in gebrauchsfertigem Zustand bereit gestellt werden. Zur Abgabe eines Produkts muss lediglich der osmotische Antrieb ausgelöst werden.

Gemäß der vorliegenden Erfindung ist ein Fördermittel zur Förderung von Produkt aus der Produktkammer, bzw. aus der Ampulle, vorgesehen, das entlang einer Längsachse des Gehäuses gegenüber dem Gehäuse verschiebbar ist. Als Fördermittel eignet sich z. B. ein Kolben, der auf einen Stopfen innerhalb der Ampulle wirkt, oder direkt der Stopfen in der Ampulle. Beim Betrieb der Verabreichungsvorrichtung grenzt das Fördermittel direkt an die zweite Kammer an und kann durch den in der Kammer herrschenden Druck in Längsrichtung des Gehäuses innerhalb der Produktkammer auf einen Auslass zu verschoben werden, so dass Produkt aus der Verabreichungsvorrichtung ausgeschüttet werden kann. Durch den osmotischen Antrieb wird ein kontinuierlicher Druck auf das Fördermittel ausgeübt, so dass ein konstanter Volumenstrom des Produkts erzielt werden kann.

Mit einer Verabreichungsvorrichtung der vorliegenden Erfindung ist es möglich über einen längeren Zeitraum auch kleinste Produktvolumina kontinuierlich zu Verabreichen. Eine einmalige Abgabe z. B. von hochkonzentrierten oder langwirkenden Medikamenten, wie etwa bei einer Injektion von langwirkendem Insulin, deren Resorption von diversen Parameter abhängt und dadurch in der Entfaltung ihrer Wirkung stark schwanken kann, sind nicht erforderlich. Durch die kontinuierliche Abgabe über längere Zeit hinweg, entstehen weniger Komplikationen und Schwankungen in der Pharmakokinetik und - dynamik. Ferner ist es vorteilhaft, dass bei einer Produktverabreichung durch eine erfindungsgemäße Vorrichtung der Produktvolumenstrom jederzeit unterbrochen werden kann. Komplikationen durch eine Verabreichung von zu großen Produktdosen, die zu potentiell gefährlichen Gesundheitszuständen führen können, wie z. B. zu Hypoglykärnie bei einem zu großen Insulindepot, können vermieden werden. Bei einer Verabreichungsvorrichtung nach der vorliegenden Erfindung ist es ferner möglich, bereits vorgefüllte oder auch vom Patienten selbst befüllte Ampullen zu verwenden. Ein umständliches Befüllen des gesamten Verabreichungsgeräts ist nicht notwendig. Dabei ist es vorteilhaft, dass Reibungen, wie sie beispielsweise zwischen Ampulle und Stopfen auftreten können, durch den hohen virtuellen osmotischen Druck vernachlässigt werden können. Weiter ist die Verabreichung des Produkts von der Lage der Verabreichungsvorrichtung unabhängig, so dass diese in einfacher Weise am Körper mitgeführt werden kann, ohne dass z. B. zusätzlich elektrische Energiequellen z. B. zum Antrieb einer Pumpe zum Einsatz kommen müssen.

Bei einer ersten Ausführungsform einer Vorrichtung zum Verabreichen eines injizierbaren Produkts nach der vorliegenden Erfindung umfasst die Auslösevorrichtung das Fördermittel, d.h. das Fördermittel dient neben der Förderung von Produkt auch zum Auslösen des osmotischen Antriebs. Hierfür ist das Fördermittel vorteilhaft derart angeordnet, dass es entgegen einer Förderrichtung des Produkts gegenüber dem Gehäuse beweglich ist. Das heißt zur Förderung von Produkt aus der Produktkammer wird das Fördermittel durch den osmotischen Antrieb entlang der Längsachse der Verabreichungsvorrichtung in Förderrichtung bewegt und zum Auslösen des osmotischen Antriebs wird das Fördermittel genau entgegengesetzt zur Förderrichtung bewegt. Dabei ist das Fördermittel derart gegenüber dem unpermeablen Trennmittel angeordnet, dass es durch die Gegenförderbewegung das Trennmittel durchbrechen kann, so dass der osmotische Antrieb ausgelöst wird. Dabei ist es vorteilhaft, wenn das Fördermittel nach dem Durchbrechen des Trennmittels sich unmittelbar an die zweite Kammer mit der Lösung anschließt, so dass der Druck in der Kammer auf das Fördermittel zur Förderung des Produkts übertragen werden kann.

In dieser ersten Ausführungsform wird das Fördermittel von einem Kolben gebildet, dessen Längsachse entlang der Längsrichtung des Gehäuses angeordnet ist und der mit einem Ende in die Produktkammer ragt und mit einer Stirnseite am anderen Ende dem unpermeablen Trennmittel vorzugweise frontal gegenüberliegt. Der Kolben wird innerhalb dem Gehäuse derart geführt, dass er auf das unpermeable Trennmittel hin beweglich ist. Die Bewegung des Kolbens kann z. B. beim Einsetzen einer Ampulle in die Produktkammer erfolgen, die den Kolben entgegen der Förderrichtung in Richtung auf das Trennmittel zu bewegt. Dabei kann vorteilhafterweise gleichzeitig ein Kontakt mit einem Stopfen innerhalb der Ampulle hergestellt werden. Der Stopfen wird dann von dem Kolben innerhalb der Ampulle auf den Auslass der Produktkammer hin verschoben. Gleichzeitig mit dem Einsetzen der Ampulle und dem Auslösen des osmotischen Antriebs kann ein Priming der Verabreichungsvorrichtung erfolgen, um die Vorrichtung in eine Startposition zu bringen.

Bei einer besonders bevorzugten Ausführungsform ist die erste Kammer als Ringkammer vorgesehen. An einer Seite ist die Ringkammer abgeschlossen und an der anderen Seite grenzt sie an die zweite Kammer. Der Hohlraum der zweiten Kammer weist einen ersten Bereich, der sich über den gesamten Außendurchmesser der Ringkammer erstreckt, und einen zweiten Bereich auf, der einen kleineren Durchmesser besitzt und sich zentral durch das innere der Ringkammer bis zur Produktkammer erstreckt. Das unpermeable Trennmittel ist dabei im Übergang des Bereichs mit großem Durchmesser zu dem Bereich mit kleinem Durchmesser angeordnet, so dass es die beiden Bereiche der zweiten Kammer trennt und auch die zweite Kammer gegenüber der ersten Kammer abtrennt. Solange das unpermeable Trennmittel vorhanden ist, befindet sich die Lösung ausschließlich in dem Bereich der zweiten Kammer mit dem großen Durchmesser. In dem zylinderförmigen Bereich mit dem kleinen Durchmesser kann der Kolben angeordnet werden, der dadurch erfindungsgemäß sowohl zum Auslösen des osmotischen Antriebs entgegen der Förderrichtung auf das Trennmittel zu bewegt werden kann, als auch nach dem Auslösen des osmotischen Antriebs in Förderrichtung in die Produktkammer hinein bewegt werden kann.

Bei einer zweiten Ausführungsform der Vorrichtung zur Verabreichung eines injizierbaren Produkts nach der vorliegenden Erfindung ist die Auslösevorrichtung derart gegenüber dem Gehäuse beweglich, dass sie den Innenraum der ersten Kammer mit dem Lösungsmittel komprimieren kann. Bevorzugt ist die Auslösevorrichtung hierfür durch ein gegenüber dem Gehäuse und dem Trennmittel entlang der Längsachse verschiebbares Hülsenelement gebildet. Besonders bevorzugt ist das Hülsenelement als Hülsenkappe an einem Ende des Gehäuses vorgesehen, das einem Ende mit dem Produktauslass gegenüberliegt. Das Hülsenelement bildet dann den Abschluss für eine Stirnseite der ersten Kammer, die der Seite an welchem ein unpermeables Trennmittel angeordnet ist, gegenüberliegt. Das Trennmittel trennt einen kleinen Bereich der zweiten Kammer, an den sich die semipermeable Membran anschließt ab, der nicht mit Lösungsmittel gefüllt ist. Das Trennmittel und die semipermeable Membran liegen sich daher gegenüber, weisen jedoch einen lichten Abstand zu einander auf. Die zweite Kammer grenzt an die semipermeable Membran und wird von dieser abgeschlossen. Auf der der Membran gegenüberliegenden Seite der zweiten Kammer schließt sich ein Fördermittel zur Förderung des Produkts an. Das Fördermittel kann direkt durch einen Stopfen einer Ampulle gebildet werden, der in Längsrichtung der Ampulle auf den Auslass zu verschiebbar ist.

Zum Auslösen des osmotischen Antriebs wird die Hülsenkappe auf das Gehäuse der Verabreichungsvorrichtung gedrückt oder über ein Gewinde durch Drehen translatorisch in Förderrichtung bewegt. Durch diese Bewegung wird die erste Kammer komprimiert, wobei sich in der Kammer ein Druck aufbaut. Der Druck wirkt auf das unpermeable Trennmittel, wie etwa eine dünne Folie, so dass diese zerstört wird und das Lösungsmittel aus der ersten Kammer in Kontakt zur semipermeablen Membran tritt. Dadurch wird der osmotische Antrieb ausgelöst, die zweite Kammer mit Druck beaufschlagt und der Stopfen in der Ampulle in Förderrichtung bewegt. Um ein ungewolltes Auslösen der Auslösevorrichtung zu verhindern, kann zwischen der Hülsenkappe und dem Gehäuse ein Abstandshalter vorgesehen sein, durch den die translatorische Bewegung der Hülsenkappe entlang der Längsachse des Gehäuses blockiert wird. Zum Auslösen wird dieser Abstandshalter entfernt. Bei dieser Ausführungsform kommt das Lösungsmittel schnell über eine große Fläche direkt mit der semipermeablen Membran in Kontakt und es erfolgt eine schnelle Beaufschlagung mit dem osmotischen Druck.

Die Verabreichungsvorrichtung kann bei dieser Ausführungsform bereits mit einer Ampulle und einem Stopfen vormontiert sein und wird für den Gebrauch vorher mit einem gewünschten Produkt befüllt. Es ist aber auch möglich keine Ampulle in der Verabreichungsvorrichtung vorzusehen, und diese als Fertigteil mit Stopfen in die Verabreichungsvorrichtung je nach Erforderlichkeit einzusetzen. In diesem Fall wird die zweite Kammer mit der Lösung durch eine Abdeckung abgeschlossen, die beim Einsetzen der Ampulle entfernt werden muss, um den Kontakt des Stopfens zur zweiten Kammer zu ermöglichen.

Weiter ist es bei einer Verabreichungsvorrichtung nach der vorliegenden Erfindung vorteilhaft, eine Druckminderungsvorrichtung zur Minderung des von dem osmotischen Antrieb auf das Fördermittel ausgeübten Drucks vorzusehen. Dadurch kann der auf das Fördermittel ausgeübte Druck reguliert und damit der Volumenstrom des Produkts aus der Verabreichungsvorrichtung kontrolliert werden. Die Druckminderungsvorrichtung kann z. B. durch eine dritte Kammer gebildet werden, die zwischen der zweiten Kammer und dem Fördermittel vorgesehen ist und die über eine druckmindernde Fluidverbindung mit der zweiten Kammer verbunden ist. Beim Auslösen des osmotischen Antriebs fließt die Lösung von der zweiten Kammer durch die druckmindernde Fluidverbindung in die dritte Kammer, in der dann ein geringerer Druck als in der zweiten Kammer herrscht. Das Fördermittel schließt sich direkt an die dritte Kammer an, so dass es in Förderrichtung angetrieben wird. Die druckmindernde Fluidverbindung kann z. B. durch ein Kapillarensystem oder einen spiraligen Fluidkanal gebildet werden. Eine derartige Druckminderungsvorrichtung ist aus der deutschen Patentanmeldung DE 199 39 023 A1 der Anmelderin bekannt, auf die hiermit Bezug genommen werden soll.

Weiter kann bei der Verabreichungsvorrichtung nach der vorliegenden Erfindung eine Ausschüttkontrollvorrichtung zur Kontrolle des von der Verabreichungsvorrichtung ausgeschütteten Produkts vorgesehen sein. Hierfür kann z. B. ein einfaches Ventil verwendet werden. Besonders bevorzugt wird jedoch eine Ausschüttkontrollvorrichtung verwendet, an die das Produkt aus dem Produktbehälter abgegeben wird und bei der das Fördermittel das Produkt an einem Einlass der Ausschüttkontrollvorrichtung mit Druck beaufschlagt, wobei das Fördermittel von der Ausschüttkontrollvorrichtung entkoppelt ist. Eine solche Ausschüttkontrollvorrichtung wird in der deutschen Patentanmeldung DE 102 33 622 der Anmelderin beschrieben, auf deren Offenbarung Bezug genommen und deren Priorität beansprucht wird.

Bei der vorliegenden Erfindung kann die Ausschüttkontrollvorrichtung auch eine Einrichtung zur Einstellung der Permeabilität der semipermeablen Membran umfassen. Hierfür werden für die semipermeable Membran Materialien verwendet, deren Permeabilität sich durch elektromagnetische Parameter, wie z. B. durch die elektrische Spannung, gezielt einstellen lässt. Zur Einstellung der Permeabilität sind in der Ausschüttkontrollvorrichtung eine Elektronik und eine Energiequelle vorgesehen. Die Ausschüttkontrollvorrichtung kann somit den osmotischen Druck derart steuern, dass ein variabler Volumenstrom des Produkts generiert wird. Materialien mit einer einstellbaren Permeabilität sind z. B. aus der US 4,513,034 und der US 5,869,078 bekannt.

Mit einer Verabreichungsvorrichtung nach der vorliegenden Erfindung ist es möglich, einen kontinuierlichen konstanten oder auch variablen Volumenstrom auszubilden, der auf eine bestimmte medizinische oder therapeutische Anwendung speziell angepasst werden kann. Durch die Verwendung herkömmlicher Ampullen kann das Verabreichungsgerät in einfacher Weise für verschiedene Wirkstoffe verwendet werden und durch den erfindungsgemäß verbesserten osmotischen Antrieb wird eine schnelle und einfache Bereitstellung des Wirkstoffs sichergestellt.

Nachfolgend werden zwei bevorzugte Ausführungsformen der vorliegenden Erfindung unter Bezugnahme auf die beigefügten Figuren beschrieben. Es stellen dar:
- Figur 1: einen Längsschnitt eines Bereichs einer Verabreichungsvorrichtung in nichtausgelöstem Zustand gemäß einer ersten Ausführungsform,
- Figur 2: einen Längsschnitt der Verabreichungsvorrichtung nach Figur 1 in ausgelöstem Zustand,
- Figur 3: einen Längsschnitt eines Bereichs einer Verabreichungsvorrichtung in nichtausgelöstem Zustand gemäß einer zweiten Ausführungsform und
- Figur 4: einen Längsschnitt einer Verabreichungsvorrichtung nach Figur 3 in ausgelöstem Zustand.

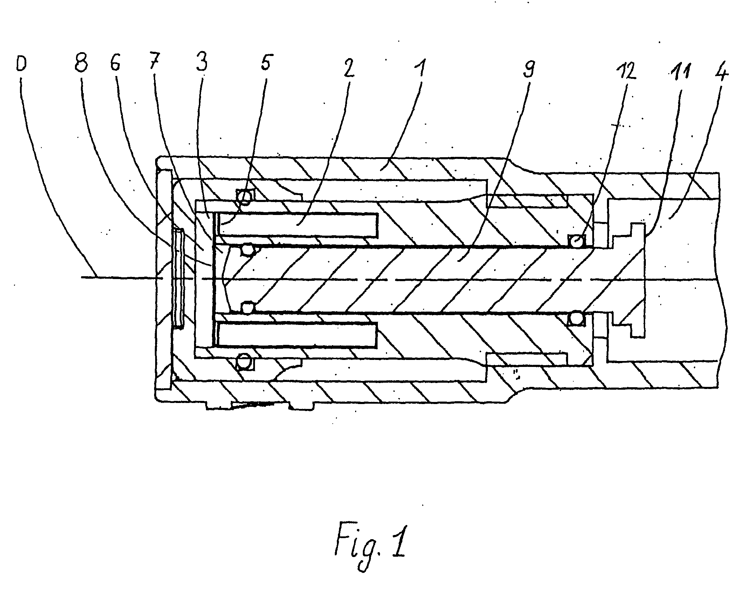

In Figur 1 ist der hintere Bereich einer Vorrichtung zur Verabreichung eines injizierbaren Produkts nach einer ersten Ausführungsform der vorliegenden Erfindung gezeigt. In einem Gehäuse 1 ist eine erste Kammer 2 mit einem Lösungsmittel, eine zweite Kammer 3 mit einer übersättigten Salzlösung und eine Produktkammer 4, in die eine in Figur 1 nicht gezeigte Ampulle mit dem Produkt eingesetzt werden kann, aufgenommen. Die erste Kammer 2 ist als Ringkammer konzentrisch um eine Längsachse D des Gehäuses 1 angeordnet. An einer Seite ist die erste Kammer 2 abgeschlossen und an einer der abgeschlossenen Seite gegenüberliegenden Seite wird die erste Kammer 2 von einer semipermeablen Membran 5 abgeschlossen. Der Hohlraum der zweiten Kammer 3 erstreckt sich in einem ersten Bereich 6 über den gesamten Außendurchmesser der ersten Kammer 2 und in einem zweiten Bereich 7, der sich an den ersten Bereich 6 anschließt, mit einem kleineren Durchmesser entlang der Längsachse D durch das Innere des Ringbereichs der ersten Kammer 2 bis er in die Produktkammer 4 mündet. Der erste Bereich 6 und der zweite Bereich 7 werden durch ein unpermeables Trennmittel in Form einer dünnen Folie 8 voneinander getrennt. Die Folie 8 erstreckt sich über die gesamte Querschnittsfläche des Bereichs 6 mit dem größeren Durchmesser und liegt mit ihrem äußeren Bereich der an der ersten Kammer 2 angebrachten semipermeablen Membran mit geringem Abstand gegenüber. Die Salzlösung befindet sich in nichtausgelöstem Zustand der Verabreichungsvorrichtung ausschließlich in dem ersten Bereich 6 der zweiten Kammer 3.

Als Fördermittel zur Förderung von Produkt aus der Produktkammer 4 ist innerhalb dem zweiten Bereich 7 der zweiten Kammer 3 ein Kolben 9 angeordnet, der sich beinahe über die gesamte Länge des zweiten Bereichs 7 erstreckt. An einem ersten Ende liegt der Kolben 9 mit seiner Stirnfläche der Folie 8 mit geringem Abstand gegenüber. Mit dem anderen Ende mündet der Kolben 9 in die Produktkammer 4 und bildet mit der diesseitigen Stirnfläche eine Antriebsfläche 11. Der Kolben 9 ist innerhalb dem zweiten Kammerbereich 7 gegenüber dem Gehäuse 1 entlang der Längsachse D beweglich angeordnet und wird durch die Form des zweiten Bereichs 7 geführt. Zwischen dem Kolben 9 und der Wand des zweiten Kammerbereichs 7 sind Dichtmittel in Form von Gummiringen 12 angeordnet, die beim Antrieb der Verabreichungsvorrichtung den Kolben gegen den zweiten Bereich 7 und das Lösungsmittel abdichten. Die Gummiringe 12 können auch zur Stabilisierung des Kolbens 9 innerhalb dem zweiten Kammerbereich 7 dienen.

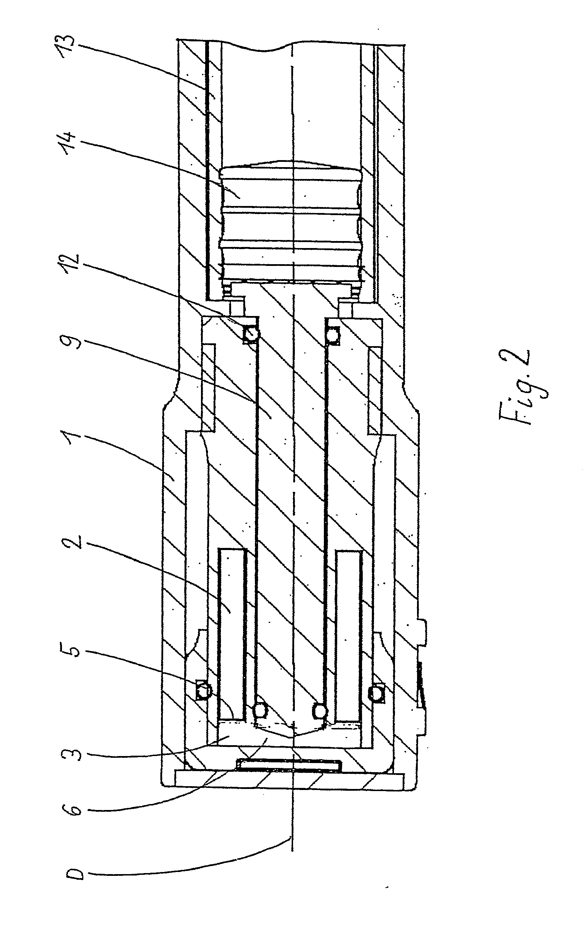

In Figur 2 ist die Verabreichungsvorrichtung nach der Ausführungsform aus Figur 1 in einem ausgelösten Zustand gezeigt. In dieser Ausführungsform wird die Auslösevorrichtung zum Auslösen des osmotischen Antriebs von der unpermeablen Folie 8 und dem entlang der Längsachse D des Gehäuses 1 verschiebbaren Kolben 9 gebildet. Zum Auslösen der Verabreichungsvorrichtung wird der Kolben 9 entgegen der Förderrichtung des Produkts in Richtung auf die Folie 8 hin bewegt bis er diese durchbricht. Der Kolben 9 wird durch das Einsetzen einer Ampulle 13 in die Produktkammer 4 in Richtung auf die Folie 8 bewegt. Beim Einsetzen der Ampulle 13 stößt ein Stopfen 14 gegen die Antriebsfläche 11 des Kolbens 9. Ein Auslass der Ampulle, der dem Stopfen 14 gegenüberliegt (nicht gezeigt), ist zunächst verschlossen, so dass mittels des Angriffs am Stopfen 14 der Kolben 9 verschoben werden kann.

Durch das Durchbrechen der unpermeablen Folie 8 gelangt Lösungsmittel an die semipermeable Membran 5, so dass die osmotische Wirkung zwischen der Lösung und dem Lösungsmittel einsetzt. Der dabei in der zweiten Kammer 3 erzeugte osmotische Druck treibt den Kolben 9 entlang der Längsachse D in Förderrichtung gegen den Stopfen 14 an, wobei die Lösung in den zweiten Bereich 7 der zweiten Kammer 3 vordringt. Nach dem Öffnen des Auslasses der Ampulle kann Produkt aus der Ampulle 13 mittels des angetriebenen Stopfens 14 ausgeschüttet werden. Die Ampulle selbst kann dabei z. B. durch einen Adapter im Gehäuse gehalten werden. An dem Adapter kann z. B. ein mit einer Durchstechnadel versehener Anschluss oder ein Luer vorgesehen sein, die bzw. der mit dem Auslass verbunden ist und das Produkt an eine gewünschte Stelle leitet. Der erzeugte Volumenstrom kann durch Auswahl des Membranmaterials sowie der Membrangeometrie an die bestimmten Anforderungen einer medizinischen oder therapeutischen Anwendung angepasst werden. Die erste Kammer 2 mit dem Lösungsmittel ist mit porösem Material, wie z. B. einem Vliess, ausgelegt, welches durch Kapillarkräfte ständig Lösungsmittel an die semipermeable Membran 5 heranführt. Dadurch wird eine Lageunabhängigkeit der Verabreichungsvorrichtung gewährleistet.

Durch das Prinzip der umgekehrten Osmose (reverse osmose) ist der osmotische Vorgang zur Druckerzeugung beim Antrieb der Verabreichungsvorrichtung umkehrbar. Die Verabreichungsvorrichtung kann daher wiederverwendbar ausgestaltet werden.

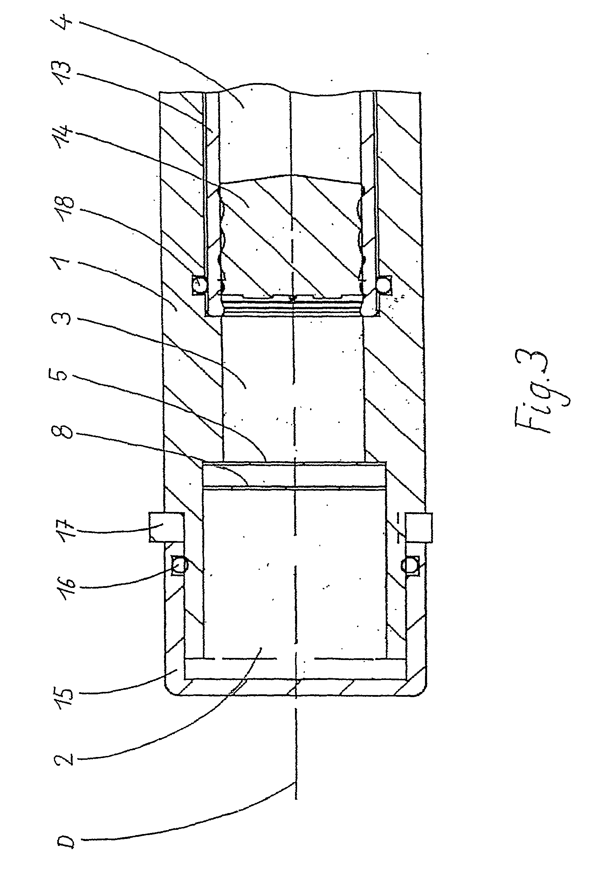

In Figur 3 ist eine Teilansicht einer Verabreichungsvorrichtung nach einer zweiten Ausführungsform der vorliegenden Erfindung gezeigt In dem Gehäuse 1 ist eine erste Kammer 2, eine zweite Kammer 3 und eine Produktkammer 4 aufgenommen. In die Produktkammer 4 ist bereits eine Ampulle 13 mit einem Stopfen 14 eingesetzt. Als Auslösevorrichtung ist bei dieser Ausführungsform eine gegenüber dem Gehäuse 1 entlang der Längsachse D des Gehäuses 1 verschiebbare Hülsenkappe 15 vorgesehen. Die Hülsenkappe 15 wird von Gummiringen 16 gegenüber dem Gehäuse abgedichtet und an diesem gehalten. Die Hülsenkappe 15 ist durch einen Abstandshalter 17 in einer ersten Position gegenüber dem Gehäuse gehalten, in der die Verabreichungsvorrichtung in einem nichtausgelösten Zustand ist.

Die Hülsenkappe 15 begrenzt die erste Kammer 2 mit dem Lösungsmittel zu einer Seite. Auf der gegenüberliegenden Seite ist die erste Kammer 2 mit einer unpermeablen Trennfolie 8 versehen, die einen Bereich der Kammer abtrennt, der der semipermeablen Membran 5 gegenüberliegt. Zwischen der unpermeablen Trennfolie 8 und der semipermeablen Membran 5 besteht ein lichter Abstand, so dass das Lösungsmittel in der ersten Kammer 2 nicht mit der semipermeablen Membran 5 in Kontakt tritt. Die semipermeable Membran schließt die zweite Kammer 3 an der Seite ab, die der ersten Kammer 2 zugewandt ist.

Auf der der semipermeablen Membran 5 gegenüberliegenden Seite wird die zweite Kammer 3 durch den Stopfen 14 abgeschlossen. Der Stopfen 14 ist daher in direktem Kontakt mit der übersättigten Salzlösung in der zweiten Kammer 3. Der Stopfen 14 wirkt beim Betrieb der Verabreichungsvorrichtung als Fördermittel zur Förderung des Produkts aus der Ampulle 13. Die Abdichtung zwischen der Ampulle 13 und dem Gehäuse 1 erfolgt über einen Gummiring 18.

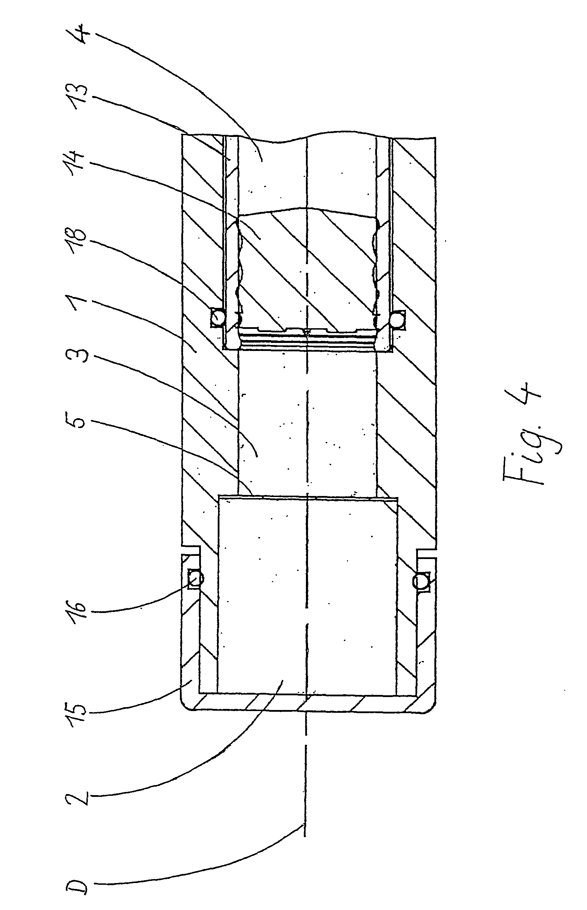

In Figur 4 ist die Verabreichungsvorrichtung aus Figur 3 in einem ausgelösten Zustand gezeigt. Der Abstandshalter 17 aus Figur 3 wurde entfernt und die Hülsenkappe 15 entlang der Längsachse D des Gehäuses 1 in Richtung auf das Gehäuse zu verschoben bis sie an einer Kante des Gehäuses anstößt. Dabei übt die Hülsenkappe 15 einen Druck auf die unpermeable Folie 8 aus, da sie die zweite Kammer 3 komprimiert. Durch den ausgeübten Druck platzt die unpermeable Folie 8 und gibt für das Lösungsmittel den Zugang zur semipermeablen Membran 5 frei. Dadurch wird der osmotische Vorgang aktiviert und in der zweiten Kammer 3 wird ein osmotischer Druck aufgebaut, der unmittelbar auf den Stopfen 14 in der Ampulle 13 wirkt. Der Stopfen 14 wird in Förderrichtung angetrieben und verdrängt das Produkt aus der Ampulle 13 durch einen dem Stopfen 14 gegenüberliegenden Auslass (nicht gezeigt).

Grundsätzlich ist es auch möglich an der Innenseite der Hülsenkappe, die der unpermeablen Folie 8 gegenüberliegt ein spitzes Element vorzusehen, dass von der Hülseninnenseite in Richtung der Folie 8 abragt und kurz vor dieser endet. Beim Aufdrücken der Hülsenkappe 15 auf das Gehäuse kann das spitze Element die Folie 8 durchtrennen und den Auslösevorgang zum Auslösen des osmotischen Antriebs unterstützen.

### Bezugszeichen:

- 1: Gehäuse
- 2: erste Kammer
- 3: zweite Kammer
- 4: Produktkammer
- 5: semipermeable Membran
- 6: erster Bereich
- 7: zweiter Bereich
- 8: Folie
- 9: Kolben
- 10: Stirnfläche
- 11: Antriebsfläche
- 12: Gummiring
- 13: Ampulle
- 14: Stopfen
- 15: Hülsenkappe
- 16: Gummiring
- 17: Abstandshalter
- 18: Gummiring
- D: Längsachse

## Patentansprüche

1. Vorrichtung zur Verabreichung eines injizierbaren Produkts mit einem osmotischen Antrieb, umfassend:
a) ein Gehäuse (1) in dem aufgenommen ist:
- eine erste Kammer (2) mit einem Lösungsmittel
- eine zweite Kammer (3) mit einer Lösung und
- eine Produktkammer (4) zur Aufnahme des Produkts,
b) eine semipermeable Membran (5), die zwischen der ersten Kammer (2) und der zweiten Kammer (3) angeordnet ist,
c) ein Fördermittel (9; 14) zur Förderung von Produkt aus der Produktkammer (4), wobei das Fördermittel (9; 14) entlang einer Längsachse (D) des Gehäuses (1) gegenüber dem Gehäuse (1) verschiebbar ist
d) eine Auslösevorrichtung (9; 15) zum Auslösen des osmotischen Antriebs mit einem unpermeablen Trennmittel (8) zwischen der ersten Kammer (2) und der zweiten Kammer (3),
**dadurch gekennzeichnet, dass**
e) die erste Kammer (2) mit einem porösen Material gefüllt ist.

2. Verabreichungsvorrichtung nach Anspruch 1, **dadurch gekennzeichnet, dass** die Auslösevorrichtung das Fördermittel (9) umfasst.

3. Verabreichungsvorrichtung nach Anspruch 1 oder 2, **dadurch gekennzeichnet, dass** das Fördermittel (9; 14) zum Auslösen des osmotischen Antriebs entgegen einer Förderrichtung des Produkts gegenüber dem Gehäuse (1) bewegbar ist.

4. Verabreichungsvorrichtung nach einem der vorhergehenden Ansprüche, **dadurch gekennzeichnet, dass** das Fördermittel (9) zum Durchbrechen des unpermeablen Trennmittels (8) vorgesehen ist.

5. Verabreichungsvorrichtung nach einem der vorhergehenden Ansprüche, **dadurch gekennzeichnet, dass** das Fördermittel von einem Kolben (9) gebildet wird, der innerhalb der zweiten Kammer (3) geführt wird.

6. Verabreichungsvorrichtung nach einem der vorhergehenden Ansprüche, **dadurch gekennzeichnet, dass** die erste Kammer (2) als Ringkammer um die zweite Kammer (3) angeordnet ist.

7. Verabreichungsvorrichtung nach einem der vorhergehenden Ansprüche, **dadurch gekennzeichnet, dass** das Fördermittel (9) durch Einsetzen eines Produktbehälters (13) in die Produktkammer (4) entgegen der Förderrichtung bewegt wird.

8. Verabreichungsvorrichtung nach Anspruch 1, **dadurch gekennzeichnet, dass** die Auslösevorrichtung (15) die erste Kammer (2) komprimiert.

9. Verabreichungsvorrichtung nach dem vorhergehenden Anspruch, **dadurch gekennzeichnet, dass** die Auslösevorrichtung (15) durch ein gegenüber dem Gehäuse (1) und dem Trennmittel (8) entlang der Längsachse (D) verschiebbares Hülsenelement (15) gebildet ist.

10. Verabreichungsvorrichtung nach einem der vorhergehenden Ansprüche, **dadurch gekennzeichnet, dass** eine Druckminderungsvorrichtung zur Minderung des von dem osmotischen Antrieb auf das Fördermittel (9; 14) ausgeübten Drucks vorgesehen ist.

11. Verabreichungsvorrichtung nach dem vorhergehenden Anspruch, **dadurch gekennzeichnet, dass** zwischen der zweiten Kammer (3) und dem Fördermittel (9; 14) eine dritte Kammer vorgesehen ist, an die das Fördermittel (9; 14) anschließt und die über eine druckmindernde Fluidverbindung mit der zweiten Kammer (3) verbunden ist.

12. Verabreichungsvorrichtung nach einem der beiden vorhergehenden Ansprüche, **dadurch gekennzeichnet, dass** die druckmindernde Fluidverbindung durch ein kapillaren System oder einen spiraligen Fluidkanal gebildet ist.

13. Verabreichungsvorrichtung nach einem der vorhergehenden Ansprüche, **dadurch gekennzeichnet, dass** eine Ausschüttkontrollvorrichtung zur Kontrolle des von der Verabreichungsvorrichtung ausgeschütteten Produkts vorgesehen ist.

14. Verabreichungsvorrichtung nach dem vorhergehenden Anspruch, **dadurch gekennzeichnet, dass** das Fördermittel (9; 14) das Produkt an einem Einlass der Ausschüttkontrollvorrichtung mit Druck beaufschlagt und das Fördermittel von der Ausschüttkontrollvorrichtung entkoppelt ist.

15. Verabreichungsvorrichtung nach einem der vorhergehenden Ansprüche, **dadurch gekennzeichnet, dass** eine Einrichtung zur Einstellung der Permeabilität der semipermeablen Membran vorgesehen ist.

## Claims

1. A device for administering an injectable product with an osmotic drive, comprising:
a) a casing (1) in which
- a first chamber (2) comprising a solvent,
- a second chamber (3) comprising a solution, and
- a product chamber (4) for accommodating the product
are accommodated;
b) a semi-permeable membrane (5) arranged between the first chamber (2) and the second chamber (3);
c) a conveying means (9; 14) for conveying product from the product chamber (4), wherein the conveying means (9; 14) can be shifted relative to the casing (1) along a longitudinal axis (D) of the casing (1);
d) a triggering device (9; 15) for triggering the osmotic drive, comprising an impermeable separating means (8) between the first chamber (2) and the second chamber (3);
**characterised in that**
e) the first chamber (2) is filled with a porous material.

2. The administering device according to claim 1, **characterised in that** the triggering device comprises the conveying means (9).

3. The administering device according to claim 1 or 2, **characterised in that** the conveying means (9; 14) for triggering the osmotic drive can be moved relative to the casing (1) counter to a conveying direction of the product.

4. The administering device according to any one of the preceding claims, **characterised in that** the conveying means (9) is provided for piercing the impermeable separating means (8).

5. The administering device according to any one of the preceding claims, **characterised in that** the conveying means is formed by a piston (9) which is guided within the second chamber (3).

6. The administering device according to any one of the preceding claims, **characterised in that** the first chamber (2) is arranged as an annular chamber around the second chamber (3).

7. The administering device according to any one of the preceding claims, **characterised in that** the conveying means (9) is moved counter to the conveying direction by inserting a product container (13) into the product chamber (4).

8. The administering device according to claim 1, **characterised in that** the triggering device (15) compresses the first chamber (2).

9. The administering device according to the preceding claim, **characterised in that** the triggering device (15) is formed by a sleeve element (15) which can be shifted along the longitudinal axis (D) relative to the casing (1) and the separating means (8).

10. The administering device according to any one of the preceding claims, **characterised in that** a pressure reducing device is provided for reducing the pressure exerted by the osmotic drive on the conveying means (9; 14).

11. The administering device according to the preceding claim, **characterised in that** a third chamber is provided between the second chamber (3) and the conveying means (9; 14), to which the conveying means (9; 14) is connected and which is connected to the second chamber (3) via a pressure-reducing fluid connection.

12. The administering device according to any one of the preceding two claims, **characterised in that** the pressure-reducing fluid connection is formed by a capillary system or a spiral fluid channel.

13. The administering device according to any one of the preceding claims, **characterised in that** a delivery control device is provided for controlling the product delivered by the administering device.

14. The administering device according to the preceding claim, **characterised in that** the conveying means (9; 14) pressurises the product at an inlet of the delivery control device, and the conveying means is decoupled from the delivery control device.

15. The administering device according to any one of the preceding claims, **characterised in that** a means for setting the permeability of the semi-permeable membrane is provided.

## Revendications

1. Dispositif d'administration d'un produit injectable à entraînement osmotique comprenant :
a) un boîtier (1) dans lequel sont logées :
- une première chambre (2) contenant un solvant
- une deuxième chambre (3) contenant une solution et
- une chambre de produit (4) destinée à recevoir le produit,
b) une membrane semi-perméable (5) disposée entre la première chambre (2) et la deuxième chambre (3),
c) un moyen de transport (9 ; 14) destiné au transport de produit depuis la chambre de produit (4), le moyen de transport (9 ; 14) étant mobile par rapport au boîtier (1) le long d'un axe longitudinal (D) du boîtier (1),
d) un dispositif de déclenchement (9 ; 15) destiné à déclencher l'entraînement osmotique avec un moyen de séparation imperméable (8) entre la première chambre (2) et la deuxième chambre (3),
**caractérisé en ce que**
e) la première chambre (2) est remplie d'une matière poreuse.

2. Dispositif d'administration selon la revendication 1, **caractérisé en ce que** le dispositif de déclenchement comprend le moyen de transport (9).

3. Dispositif d'administration selon la revendication 1 ou 2, **caractérisé en ce que** le moyen de transport (9 ; 14) peut être déplacé par rapport au boîtier (1) pour déclencher l'entraînement osmotique en sens inverse d'une direction de transport du produit.

4. Dispositif d'administration selon l'une quelconque des revendications précédentes, **caractérisé en ce que** le moyen de transport (9) est prévu pour transpercer le moyen de séparation imperméable (8).

5. Dispositif d'administration selon l'une quelconque des revendications précédentes, **caractérisé en ce que** le moyen de transport est constitué d'un piston (9) qui est guidé à l'intérieur de la deuxième chambre (3).

6. Dispositif d'administration selon l'une quelconque des revendications précédentes, **caractérisé en ce que** la première chambre (2) est disposée comme chambre annulaire autour de la deuxième chambre (3).

7. Dispositif d'administration selon l'une quelconque des revendications précédentes, **caractérisé en ce que** le moyen de transport (9) est déplacé en sens inverse de la direction de transport par insertion d'un récipient de produit (13) dans la chambre de produit (4).

8. Dispositif d'administration selon la revendication 1, **caractérisé en ce que** le dispositif de déclenchement (15) comprime la première chambre (2).

9. Dispositif d'administration selon la revendication précédente, **caractérisé en ce que** le dispositif de déclenchement (15) est constitué d'un élément manchon (15) mobile le long de l'axe longitudinal (D) par rapport au boîtier (1) et au moyen de séparation (8).

10. Dispositif d'administration selon l'une quelconque des revendications précédentes, **caractérisé en ce qu'**un dispositif de réduction de pression est prévu pour réduire la pression exercée par l'entraînement osmotique sur le moyen de transport (9 ; 14).

11. Dispositif d'administration selon la revendication précédente, **caractérisé en ce qu'**entre la deuxième chambre (3) et le moyen de transport (9 ; 14) est prévue une troisième chambre à laquelle est contigu le moyen de transport (9 ; 14) et laquelle est reliée à la deuxième chambre (3) par l'intermédiaire d'une liaison fluidique réduisant la pression.

12. Dispositif d'administration selon l'une quelconque des deux revendications précédentes, **caractérisé en ce que** la liaison fluidique réduisant la pression est constituée d'un système capillaire ou d'un canal fluidique en spirale.

13. Dispositif d'administration selon l'une quelconque des revendications précédentes, **caractérisé en ce qu'**un dispositif de contrôle de déversement est prévu pour contrôler le produit déversé par le dispositif d'administration.

14. Dispositif d'administration selon la revendication précédente, **caractérisé en ce que** le moyen de transport (9 ; 14) met le produit sous pression à une entrée du dispositif de contrôle de déversement, et le moyen de transport est désaccouplé du dispositif de contrôle de déversement.

15. Dispositif d'administration selon l'une quelconque des revendications précédentes, **caractérisé en ce qu'**un dispositif destiné à régler la perméabilité de la membrane semi-perméable est prévu.
